# EUROPÄISCHE PATENTSCHRIFT

(11) **EP 3 758 763 B1**
(45) Veröffentlichungstag und Bekanntmachung des Hinweises auf die Patenterteilung: **07.06.2023**
(21) Anmeldenummer: 19712906.7
(22) Anmeldetag: 04.03.2019
(51) Int. Cl.: A61L 27/10

(54) **OHRIMPLANTAT**
EAR IMPLANT
IMPLANT AUDITIF

(30) Priorität: 02.03.2018 DE 102018104839
(43) Veröffentlichungstag der Anmeldung: 06.01.2021
(73) Patentinhaber: caput medical GmbH, 40822 Mettmann (DE)
(72) Erfinder: SUDHOFF, Holger, 33604 Bielefeld (DE); HÜTTER, Friedmar, 40489 Düsseldorf (DE)
(74) Vertreter: Schneiders & Behrendt Bochum
(86) Internationale Anmeldenummer: PCT/EP2019/055246
(87) Internationale Veröffentlichungsnummer: WO 2019/166663

(56) Entgegenhaltungen:
- WO-A1-00/41651
- WO-A1-2017/205596
- WO-A2-00/34196
- WO-A2-2009/140362
- WO-A2-2013/053866
- DE-U1-202007 017 910

## Beschreibung

Die Erfindung betrifft Ohrimplantate zur Verbesserung oder Wiederherstellung der Hörfähigkeit bei Defekten im Bereich der Ohrknöchelchen oder der Gehörgangshinterwand sowie ein Verfahren zu ihrer Herstellung.

Das menschliche Ohr übermittelt Schallwellen durch den Gehörgang auf das Trommelfell, dessen Schwingungen über die Gehörknöchelchen des Mittelohrs zum ovalen Fenster und in die Hörschnecke weitergeleitet werden. Erst in der Hörschnecke werden die Schwingungen in Nervenimpulse umgewandelt, die im Gehirn verarbeitet werden. Die Schwingungsübertragung im Mittelohr erfolgt über die Gehörknöchelchen, die als Hammer, Amboss und Steigbügel bezeichnet werden, wobei der Hammer am Trommelfell anliegt und der Steigbügel am ovalen Fenster. Die Hörfähigkeit hängt entscheidend von der Funktion und dem Zusammenwirken der Gehörknöchelchen ab, insbesondere von deren Fähigkeit, mechanische Schwingungen vom Trommelfell zum ovalen Fenster weiterzuleiten.

Sobald die Schwingungsübertragung im Bereich der Gehörknöchelchen gestört ist, tritt eine Beeinträchtigung der Schallwahrnehmung ein. Solche Störungen können angeboren sein, beispielsweise bei Defekten oder Fehlen eines oder mehrerer Gehörknöchelchen, oder durch Krankheit oder Verletzung bedingt sein. Abnehmendes Gehör ist eine weitverbreitete Alterserscheinung.

Eine typische Erkrankung, die zum vollständigen oder teilweisen Verlust des Gehörs führen kann, ist die Otosklerose, eine Erkrankung des menschlichen Felsenbeins, des Knochens, der das Innenohr umgibt. Die Krankheit führt zu einer Fixierung des normalerweise locker schwingenden Steigbügels, der damit das Schallsignal nicht mehr auf das ovale Fenster und damit in das Innenohr übertragen kann. Die Otosklerose wird in der Regel operativ durch Einsetzen einer Prothese behandelt.

Bei der Behandlung von Defekten im Mittelohr muss zwischen verschiedenen Konstellationen unterschieden werden. Im Einzelnen:
- Der Amboss oder ein Teil des Ambosses fehlt;
- Nur der Steigbügel ist erhalten;
- Der Hammer oder Hammergriff ist erhalten, während Amboss und der Oberteil des Steigbügels fehlen;
- Alle Gehörknöchelchen mit Ausnahme der Fußplatte des Steigbügels sind defekt.

In allen diesen Fällen kommt speziell angepasste Prothetik zum Einsatz, die der Überbrückung defekter oder fehlender Teile dient oder aber das Trommelfell unmittelbar und direkt mit dem ovalen Fenster oder der erhaltenen Fußplatte des Steigbügels verbindet. Die Konstellationen werden mit PORP (partial ossicular replacement prosthesis) und TORP (total ossicular replacement prosthesis) bezeichnet.

Bei der PORP wird eine Prothese implantiert, die den Defekt unter Ausnutzung der intakten Teile der Gehörknöchelchen überbrückt. Dies ist beispielsweise der Fall bei Defekten im Bereich des Ambosses, bei dem das Implantat auf den Kopf des Steigbügels aufgesetzt wird und den Kontakt zu einem intakten Teil des Ambosses oder zum Hammer herstellt.

Bei der TORP wird das Implantat auf die Fußplatte des Steigbügels oder unmittelbar auf das ovale Fenster aufgesetzt und stellt den Kontakt zum Trommelfell her. Werden die Membranen durch das Implantat unmittelbar verbunden, verfügt das Implantat über entsprechend ausgestaltete Kopf- und Fußplatten, die am Trommelfell und am ovalen Fenster anliegen.

Als Materialien für solche Implantate wurden bislang Knochen, Kunststoffe, keramische Materialien und Metalle vorgeschlagen. Bei den Kunststoffen waren dies Vinyl-Acryl-Polymerisate, PTFE und HDPE. Bei den keramischen Materialien waren dies insbesondere Aluminiumoxidkeramiken und Hydroxylapatit, ein knochenähnliches Material. Geeignete Metalle sind rostfreier Stahl und Titan. Das 1993 erstmalig eingeführte Titan hat zunehmend Marktanteile gewonnen.

Keramische Materialien und Metalle haben aufgrund ihrer Fähigkeit, mechanische Schwingungen ohne große Verluste weiterzuleiten, einen Vorteil gegenüber Kunststoffen. Tatsächlich erwiesen sich die frühen Kunststoffe und Stahlimplantate als wenig standfest; trotz guter Anfangsergebnisse waren die Langzeitergebnisse enttäuschend. Insgesamt haben sich über die Jahre keramische Materialien, Titan und Titanlegierungen durchgesetzt.

Titan und Titanlegierungen führen allerdings immer wieder zur Knochenneubildung im Implantatbereich (Osteoinduktion), die mit einer Minderung der Schwingungsübertragung einhergeht. Diese Osteoinduktion tritt insbesondere dort auf, wo das Implantat mit körpereigenem Knochenmaterial in Kontakt kommt. Zudem hat Titan den Nachteil, dass es nicht an den Implantationsort anpassbar ist, da die Härte des Materials eine kurzfristige Bearbeitung (Abschleifen, Abschneiden) ausschließt. Ein Vorteil von Titan ist seine Festigkeit und Formbarkeit/Bearbeitbarkeit auch zu sehr feinen Strukturen.

Andere Materialien, auch solche mit Apatit-Strukturen, weisen eine erhöhte Tendenz zur sekundären Ossifikation auf, die auf Dauer zu einer Verminderung der Schwingungsübertragung führt.

Ein Mittelohr-Totalimplantat (TORP) ist beispielsweise aus der DE 20 2007 017 910 U1 bekannt. Dieses Implantat ist zur mechanischen Verbindung mit der Steigbügelfußplatte vorgesehen und weist an seinem trommelfellseitigen Ende eine Kontaktplatte auf.

Die EP 1 143 881 B1 beschreibt eine Teilprothese für die Verbindung der Steigbügelfußplatte mit dem Amboss. Es handelt sich um ein Implantat nach dem PORP Prinzip.

Aus der WO 2017/205596 A1 ist eine Lithium-Disilikat-Glaskeramik bekannt, die zur Erzeugung einer Apatitphase Calciumoxid und Fluorid enthält.

Der Erfindung liegt die Aufgabe zugrunde, ein Mittelohr-Implantat bereit zu stellen, welches bei Defekten im Bereich der Gehörknöchelchen eine gute Signalübermittlung ermöglicht, eine hohe Standdauer aufweist, körperverträglich ist und insbesondere an die beim Patienten gegebene physische Situation schnell und einfach anpassbar ist. Es soll zudem auch die Ausbildung feiner Strukturen ermöglichen, wie dies bei Titan der Fall ist. Schließlich soll das Implantatmaterial eine geringe Tendenz zur sekundären Ossifikation aufweisen.

Diese Aufgabe wird mit einem Ohrimplantat der eingangs genannten Art gelöst, welches aus einer Lithium-Disilikat-Glaskeramik mit einem Molverhältnis von SiO₂ zu Li₂O von 2 bis 3 besteht, wobei die Glaskeramik mit P₂O₅ und ZrO₂ dotiert und stabilisiert ist, frei von Calciumionen ist und kein Natrium und Fluor enthält.

Lithium-Disilikat-Glaskeramik hat sich in der Dentaltechnik bewährt und wird vielfach für Zahnkronen eingesetzt. Das Material ist hart, dauerhaft, weitgehend inert, gut körperverträglich und gleicht in seinem Aussehen natürlichem Zahnmaterial. Anfänglichen Tendenzen zur Devitrifikation wurde durch Zumischen anderer Oxide begegnet. Diese weiteren Oxide bilden stabilisierende Kristallphasen, die sich mit der Hauptkristallphase aus Li₂Si₂O₅ mischen. Beispiele für solche Glaskeramiken sind in den WO 2013/053863, 864, 865 und 866 niedergelegt.

Die erfindungsgemäßen Ohrimplantate sind von üblichem Design, unterscheiden sich also von an und für sich bekannten Ohrimplantaten durch die Verwendung eines neuen Materials. Das gesamte Ohrimplantat besteht aus der Lithium-Disilikat-Glaskeramik. Es ist vollständig aus der Lithium-Disilikat-Glaskeramik gefertigt. Erforderlichenfalls können auch Defekte der hinteren Gehörgangswand, wie beispielsweise nach deren Wegnahme im Rahmen von ohrchirurgischen Eingriffen durch die Lithium-Disilikat-Glaskeramik abgedeckt werden.

Bei dem erfindungsgemäßen Ohrimplantat kann es sich um eine Teilprothese (PORP), eine Totalprothese (TORP) oder eine Rekonstruktion der hinteren Gehörgangswand handeln. Eine Totalprothese ist zur Platzierung auf der Steigbügelfußplatte mit einem Fußelement ausgestattet und weist an der Kopfseite ihres Schafts eine Kontaktplatte zur Anlage an dem Trommelfell eines Patienten auf. Anstelle eines Fußelements, das zur Platzierung auf der Steigbügelfußplatte bestimmt ist, kann auch eine Kontaktplatte zur Anlage am ovalen Fenster vorgesehen sein.

Auch bei Teilprothesen (PORP) kommen mehrere Varianten in Frage, je nach Defekt im Bereich des Mittelohrs, der auszugleichen ist. Ein häufig gebrauchtes Implantat ist dazu bestimmt auf dem Kopf eines intakten Steigbügels platziert zu werden und weist für diesen Zweck eine Aufnahme auf, die beispielsweise tassen- oder glockenförmig ausgebildet ist. Ein mehr oder weniger langer Schaft überbrückt die Lücke zum nächstgelegenen intakten Gehörknöchelchen, beispielsweise einem Teil des Amboss oder zum Hammer. Auch hier kann der Schaft an seinem Kopfende eine Kontaktplatte aufweisen, die unmittelbar am Trommelfell anliegt. In diesem Fall wird ein direkter Kontakt zwischen Steigbügel und Trommelfell hergestellt.

Die erfindungsgemäße verwandte Lithium-Disilikat-Glaskeramik ist eine stabilisierte Glaskeramik, die vorzugsweise mit P₂O₅ dotiert ist. Die Stabilisierung erfolgt durch zusätzliche Kristallphasen weiterer Metalloxide, die beispielsweise als Phosphate vorliegen können. Als weitere Metalloxide kommen insbesondere K₂O und ZnO in Frage, aber auch Al₂O₃, ebenso die Mischungen davon.

Vorzugsweise liegt das Molverhältnis von SiO₂ zu Li₂O im Bereich von 2,3 bis 2,5. Der Gehalt an ZrO₂ ist kleiner als 1,2 Gew.-% und liegt insbesondere zwischen 0,4 und 1,0 Gew.-%.

P₂O₅ dient als Nukleierungsmittel und ist mit bis zu 5 Gew.-% in der Glaskeramik enthalten.

Es versteht sich, dass alle Gewichtsangaben auf das Gesamtgewicht der Glaskeramik bezogen sind.

Die Lithium-Disilikat-Glaskeramik ist inert, chemisch stabil und langzeitbeständig und hat ein ausgezeichnetes Schwingungsübertragungsvermögen. Es lässt sich nahezu beliebig formen und ist gut anpassbar und bearbeitbar. Es kann vor Gebrauch auch bei einer Operation, kurzfristig in die jeweils vom Patienten benötigte Form und Länge gebracht werden. Zudem hat sich gezeigt, dass eine sekundäre Ossifikation weitgehend unterbleibt.

Insbesondere ist die besondere Eignung der erfindungsgemäßen Implantate auf die Abwesenheit, abgesehen von unvermeidlichen Verunreinigungen, von Calciumionen zurückzuführen. Die Glaskeramik enthält kein knochenähnliches Material mit Apatitstruktur, vor allem keinen Apatit, der die Ossifikation fördert, ebenso wenig Wollastonit. Sie enthält ferner kein Natrium und kein Fluor.

Das Implantat kann beispielsweise durch ein geeignetes Einbringen ohne weitere Fixierung auf den Steigbügelkopf oder die Steigbügelfußplatte gesetzt werden.

Die Erfindung betrifft ferner ein Verfahren zur Herstellung der Ohrimplantate.

Es versteht sich, dass erfindungsgemäße Ohrimplantate auf übliche Weise hergestellt werden können, beispielsweise durch Ausfräsen des Implantats aus einem Grünling aus Lithium-Disilikat und anschließendes Brennen des ausgefrästen Ohrimplantats. Bevorzugt und besonders geeignet ist allerdings das nachstehend beschriebene Verfahren.

Entsprechend betrifft die Erfindung ein Verfahren zur Herstellung eines Implantats, wie vorstehend beschrieben, mit den Schritten
(a) Herstellen eines Kunststoffmodells durch 3D-Druck,
(b) Einbetten des Kunststoffmodells in eine feuerfeste Masse,
(c) Brennen der feuerfesten Masse mit dem eingebetteten Kunststoffmodell unter Eliminierung des Kunststoffmodells,
(d) Einpressen einer Lithium-Disilikat-Masse in erweichtem oder pastöser Form und
(e) nach dem Abkühlen und Ausformen Ätzen und/oder Polieren des ausgeformten Implantats.

Die Herstellung des Kunststoffmodells durch 3D-Druck erlaubt - neben der individuellen Fertigung des Implantats - eine schnelle Überprüfung des Produkts auf seine Form und gegebenenfalls auch das Einpassen des Kunststoffmodells in das Mittelohr eines Patienten zur Überprüfung der Passgenauigkeit.

Für die Herstellung des Kunststoffmodells können handelsübliche 3D-Drucker verwandt werden, beispielsweise solche der Firma W2P Engineering GmbH. Es handelt sich dabei um 3D-Drucker, die die Härtung der ausgebrachten Kunststoffmasse durch Licht erlauben. Geeignete Kunststoffe sind beispielsweise lichthärtende Kunststoffe auf Methacrylatbasis der Marke SolFlex, ebenfalls von der Firma W2P.

Die Kunststoffmodelle werden anschließend in eine Einbettmasse eingebracht, die das Modell - bis auf einen Zugangskanal - fest umschließen und exakt abbilden. Eine solche Einbettmasse besteht aus üblichen feuerfesten Materialien, wie sie in der Gießereitechnik verwandt werden. Besonders geeignet ist eine phosphatgebundene Einbettmasse auf Basis von Quarz oder Cristobalit.

Die Einbettmasse mit dem eingebetteten Kunststoffmodell wird anschließend in einem Brennofen bei einer Temperatur oberhalb von 800°C gebrannt. Vorzugsweise beginnt der Brennprozess mit einer Temperatur von 850°C und steigt dann kontinuierlich auf eine Temperatur von bis zu 1.000°C an. Der Brennprozess verläuft in Gegenwart von Luft oder Sauerstoff unter Normaldruck, was die rückstandslose Beseitigung des Kunststoffmodells durch Verbrennen erlaubt. In den freiwerdenden Hohlraum wird anschließend mithilfe eines Pressstempels die Lithium-Disilikat-Masse, die in erweichter oder pastöser Form vorliegt, bei der dann herrschenden Temperatur eingepresst. Dieser Schritt erfolgt vorzugsweise im Vakuum, um Lufteinschlüsse zu vermeiden. Die Verwendung eines Pressstempels ermöglicht eine relativ hohe Verdichtung der Masse, vorzugsweise auf mehr als 90 % der theoretischen Dichte des entstehenden Lithium-Disilikat-Glaskörpers.

Nach dem Abkühlen und Ausformen wird das Implantat durch Nachbehandlung gereinigt. Der Einpressstrang wird abgeschliffen und die Oberfläche von anhaftender Einbettmasse befreit, vorzugsweise durch kurzzeitiges Anätzen mit verdünnter Flusssäure und/oder mit Ultraschall. Nach gründlichem Polieren ist das Implantat gebrauchsfertig.

Das bei der Herstellung anfallende Kunststoffmodell kann beispielsweise auch mehrfach ausgedruckt werden und als Muster verwandt werden. Das Muster kann beispielsweise einem Patienten eingepasst werden, um die Passgenauigkeit zu überprüfen und eventuell Änderungen vorzunehmen. Diese Änderungen können dann erneut ausgedruckt werden und führen zu einem passgenauen Implantat. Denkbar ist auch die Herstellung einer ganzen Serie von Kunststoffmodellen, die auf die verschiedenen Größenanforderungen von Patienten abgestimmt sind und dazu dienen, ein passendes vorgefertigtes Implantat auszuwählen.

Entsprechend offenbart wird auch ein Kunststoffmodell eines erfindungsgemäßen Ohrimplantats aus Lithium-Disilikat-Glaskeramik, hergestellt im 3D-Druck gemäß Schritt (a) von Anspruch 14. Auf die gleiche Weise und dem gleichen Materialien können im Übrigen auch passgenaue Implantate für den Knochenersatz im Schädel gefertigt werden (nicht beansprucht), wie es bei Unfallopfern nach Schädelverletzungen oder nach Schädeloperationen gebraucht wird. Es wird das gleiche Material wie für die Ohrimplantate verwandt. Die Glaskeramik ist ein schlechter Wärmeleiter, was Erfrierungen im Bereich der angrenzenden Kopfhaut im Winter vermeidet.

Schließlich ist es auch möglich, die erfindungsgemäßen im 3D-Druck direkt aus Lithium-Disilikat-Pulver herzustellen und den so hergestellten Grünling zu sintern und nachzubearbeiten. Es versteht sich, dass dazu das Pulver in eine druckfähige Form gebracht werden muss, etwa durch Anteigen oder Suspendieren.

## Patentansprüche

1. Ohrimplantat zur Verbesserung oder Wiederherstellung der Hörfähigkeit bei Defekten im Bereich der Ohrknöchelchen, welches aus einer Lithium-Disilikat-Glaskeramik mit einem Molverhältnis von SiO₂ zu Li₂O von 2 bis 3 besteht, die mit P₂O₅ und ZrO₂ dotiert und stabilisiert ist, **dadurch gekennzeichnet, dass** die Glaskeramik frei von Calciumionen ist und ferner kein Natrium und Fluor enthält.

2. Ohrimplantat nach Anspruch 1, **dadurch gekennzeichnet, dass** das Lithium-Disilikat-Glaskeramik ein mit bis zu 5 Gew.-% P₂O₅ dotiertes, stabilisiertes Lithium-Disilikat ist.

3. Ohrimplantat nach Anspruch 1 oder 2, **dadurch gekennzeichnet, dass** das Molverhältnis von SiO₂ zu Li₂O im Bereich von 2,3 bis 2,5 liegt.

4. Ohrimplantat nach einem der vorstehenden Ansprüche, **dadurch gekennzeichnet, dass** die Lithium-Disilikat-Glaskeramik K₂O enthält.

5. Ohrimplantat nach einem der vorstehenden Ansprüche, **dadurch gekennzeichnet, dass** die Lithium-Disilikat-Glaskeramik weniger als 1,2 Gew.-%, insbesondere 0,4 bis 1,0 Gew.-% ZrO₂ enthält.

6. Ohrimplantat nach einem der vorstehenden Ansprüche, **dadurch gekennzeichnet, dass** die Lithium-Disilikat-Glaskeramik Übergangsmetalloxide enthält, insbesondere ZnO.

7. Ohrimplantat nach einem der vorstehenden Ansprüche, **dadurch gekennzeichnet, dass** sie als Teilprothese (PORP) zur Platzierung auf dem Kopf des Steigbügels mit einer Aufnahme für den Steigbügelkopf und einem Kontaktelement zur Herstellung einer Verbindung mit einem der anderen (Amboss oder Hammer) Gehörknöchelchen ausgebildet ist.

8. Ohrimplantat nach Anspruch 7, **dadurch gekennzeichnet, dass** das Kontaktelement eine Gabel ist.

9. Ohrimplantat nach Anspruch 7, **dadurch gekennzeichnet, dass** das Kontaktelement eine Kontaktplatte zur Anlage an das Trommelfell ist.

10. Ohrimplantat nach einem der Ansprüche 1 bis 6, **dadurch gekennzeichnet, dass** es als Totalprothese (TORP) zur Platzierung auf der Steigbügelfußplatte mit einem Fußelement, einem Schaft und einer Kontaktplatte zur Anlage an dem Trommelfell ausgebildet ist.

11. Ohrimplantat nach einem der Ansprüche 1 bis 6, **dadurch gekennzeichnet, dass** es zum Ersatz der hinteren Gehörgangshinterwand ausgebildet ist.

12. Ohrimplantat nach einem der Ansprüche 1 bis 6, **dadurch gekennzeichnet, dass** es als Teilprothese (PORP) zur Platzierung auf dem Fuß des Steigbügels mit einem Fußelement, einem Schaft und einer Gabel zur Herstellung einer Verbindung zwischen der Fußplatte des Steigbügels und einem der anderen Gehörknöchelchen (Amboss oder Hammer) dient.

13. Verfahren zur Herstellung eines Implantats nach einem der Ansprüche 1 bis 12 mit den Schritten
(a) Herstellen eines Kunststoffmodells des Implantats durch 3D-Druck,
(b) Einbetten des Modells in eine feuerfeste Masse,
(c) Brennen der feuerfesten Masse mit dem eingebetteten Modell und Eliminieren des Modells,
(d) Einpressen einer Lithium-Disilikat-Masse in pastösem oder erweichtem Zustand und
(e) Nach dem Abkühlen und Ausformen Ätzen und/oder Polieren des ausgeformten Implantats.

14. Verfahren Anspruch 13, **dadurch gekennzeichnet, dass** zur Herstellung des Kunststoffmodells ein lichthärtender Kunststoff auf Methacrylatbasis verwandt wird.

15. Verfahren nach Anspruch 13 oder 14, **dadurch gekennzeichnet, dass** die feuerfeste Masse eine Einbettmasse auf Quarz- oder Cristobalitbasis ist.

16. Verfahren nach einem der Ansprüche 13 bis 15, **dadurch gekennzeichnet, dass** die feuerfeste Masse mit dem eingebetteten Modell in Gegenwart von Luft gebrannt wird.

17. Verfahren nach einem der Ansprüche 13 bis 16, **dadurch gekennzeichnet, dass** der Brennvorgang bei einer Temperatur von größer gleich 800°C, ansteigend auf bis zu 1.000°C, durchgeführt wird.

18. Verfahren nach einem der Ansprüche 13 bis 17, **dadurch gekennzeichnet, dass** die erreichte Lithium-Disilikat-Masse im Vakuum bei einer Temperatur von mehr als 900°C in die feuerfeste Masse eingepresst wird.

19. Verfahren nach eine Ansprüche 13 bis 18, **dadurch gekennzeichnet, dass** das ausgeformte Implantat durch Ätzen mit verdünnter Flusssäure von anhaftender feuerfester Masse befreit wird.

## Claims

1. Ear implant for improving or restoring the hearing ability in the event of defects in the region of the ossicles of the ear, which consists of a lithium disilicate glass ceramic having a molar ratio of SiO₂ to Li₂O of 2 to 3, which is stabilised and doped with P₂O₅ and ZrO₂, **characterised in that** the glass ceramic material is free of calcium ions and further does not contain sodium or fluorine.

2. Ear implant according to claim 1, **characterised in that** the stabilised lithium disilicate glass ceramic is a lithium disilicate glass ceramic doped and stabilised with up to 5 wt% P₂O₅.

3. Ear implant according to claim 1 or 2, **characterised in that** the molar ratio of SiO₂ to Li₂O is in the range of 2.3 to 2.5.

4. Ear implant according to any one of the preceding claims, **characterised in that** the lithium disilicate glass ceramic contains K₂O.

5. Ear implant according to any one of the preceding claims, **characterised in that** the lithium disilicate glass ceramic contains less than 1.2 wt%, in particular 0.4 to 1.0 wt% ZrO₂.

6. Ear implant according to any one of the preceding claims, **characterised in that** the lithium disilicate glass ceramic contains transition metal oxides, in particular ZnO.

7. Ear implant according to any one of the preceding claims, **characterised in that** it is formed as a partial prosthesis (PORP) for placement on the head of the stirrup with a receptacle for the head of the stirrup and a contact element for establishing a connection with one of the other ossicles (anvil or hammer).

8. Ear implant according to claim 7, **characterised in that** the contact element has a fork shape.

9. Ear implant according to claim 7, **characterised in that** the contact element is a contact plate for making contact with the eardrum.

10. Ear implant according to any one of claims 1 to 6, **characterised in that** it is designed as a total prosthesis (TORP) for placement on the footplate of the stirrup, having a foot element, a shaft and a contact plate for making contact with the eardrum.

11. Ear implant according to any one of the claims 1 to 6, **characterised in that** it is designed to replace the posterior wall of the auditory canal.

12. Ear implant according to any one of claims 1 to 6, **characterised in that** it serves as a partial prosthesis (PORP) for placement on the foot of the stirrup, having with a foot element, a shaft and a fork for establishing a connection between the footplate of the stirrup and one of the other ossicles (anvil or hammer).

13. Method for manufacturing an implant according to any one of claims 1 to 12 having the steps of
(a) Producing a plastic model of the implant by 3D printing,
(b) Embedding the model in a refractory mass,
(c) Firing of the refractory mass with the embedded model and eliminating the model,
(d) Pressing in a lithium disilicate mass in pasty or softened form, and
(e) After cooling and removal from the mould, etching and/or polishing the formed implant.

14. Method according to claim 13, **characterised in that** a light-curing methacrylate-based plastic is used to produce the plastic model.

15. Method according to claim 13 or 14, **characterised in that** the refractory mass is a quartz-based or cristobalite-based embedding mass.

16. Method according to any one of claims 13 to 15, **characterised in that** the refractory mass with the embedded model is fired in the presence of air.

17. Method according to any one of claims 13 to 16, **characterised in that** the firing process is carried out at a temperature greater than or equal to 800°C, rising to a temperature of 1000°C.

18. Method according to any one of claims 13 to 17, **characterised in that** the obtained lithium disilicate mass is pressed under vacuum into the refractory mass at a temperature of more than 900°C.

19. Method according to any one of claims 13 to 18, **characterised in that** the implant removed from the mould is freed from adhering refractory mass by etching with diluted hydrofluoric acid.

## Revendications

1. Implant auditif pour l'amélioration ou la restauration de la capacité auditive en cas de défaillances dans la région des osselets, lequel est constitué d'une vitrocéramique de lithium-disilicate avec un rapport molaire entre SiO₂ et Li₂O de 2 à 3, qui est dopée et stabilisée avec P₂O₅ et ZrO₂, **caractérisé en ce que** la vitrocéramique est exempte d'ions de calcium et ne contient en outre pas de sodium et pas de fluor.

2. Implant auditif selon la revendication 1, **caractérisé en ce que** la vitrocéramique de lithium-disilicate est un lithium-disilicate stabilisé dopé avec jusqu'à 5 % en poids de P₂O₅.

3. Implant auditif selon la revendication 1 ou 2, **caractérisé en ce que** le rapport molaire entre SiO₂ et Li₂O se situe dans la plage de 2,3 à 2,5.

4. Implant auditif selon l'une quelconque des revendications précédentes, **caractérisé en ce que** la vitrocéramique de lithium-disilicate contient du K₂O.

5. Implant auditif selon l'une quelconque des revendications précédentes, **caractérisé en ce que** la vitrocéramique de lithium-disilicate contient moins de 1,2 % en poids, en particulier de 0,4 à 1,0 % en poids de ZrO₂.

6. Implant auditif selon l'une quelconque des revendications précédentes, **caractérisé en ce que** la vitrocéramique de lithium-disilicate contient des oxydes de métaux de transition, en particulier du ZnO.

7. Implant auditif selon l'une quelconque des revendications précédentes, **caractérisé en ce qu'**il est réalisé en tant que prothèse partielle (PORP) destinée à être placée sur la tête de l'étrier avec un logement pour la tête d'étrier et un élément de contact pour établir une liaison avec l'un des autres osselets (enclume ou marteau).

8. Implant auditif selon la revendication 7, **caractérisé en ce que** l'élément de contact est une fourche.

9. Implant auditif selon la revendication 7, **caractérisé en ce que** l'élément de contact est une plaque de contact destinée à venir en appui sur le tympan.

10. Implant auditif selon l'une quelconque des revendications 1 à 6, **caractérisé en ce qu'**il est réalisé en tant que prothèse totale (TORP) destinée à être placée sur la plaque de base d'étrier avec un élément de base, une tige et une plaque de contact destinée à venir en appui sur le tympan.

11. Implant auditif selon l'une quelconque des revendications 1 à 6, **caractérisé en ce qu'**il est réalisé pour remplacer la paroi arrière de méat auditif.

12. Implant auditif selon l'une quelconque des revendications 1 à 6, **caractérisé en ce qu'**il fait office de prothèse partielle (PORP) destinée à être placée sur la base de l'étrier avec un élément de base, une tige et une fourche pour établir une liaison entre la plaque de base de l'étrier et un des autres osselets (enclume ou marteau).

13. Procédé pour fabriquer un implant selon l'une quelconque des revendications 1 à 12 avec les étapes
(a) de fabrication d'un modèle en matière synthétique de l'implant par impression 3D,
(b) d'intégration du modèle dans une masse réfractaire,
(c) de cuisson de la masse réfractaire avec le modèle intégré et d'élimination du modèle,
(d) d'introduction par pression d'une masse de lithium-disilicate dans un état pâteux ou ramolli, et
(e) après le refroidissement et le démoulage, de décapage et/ou de polissage de l'implant démoulé.

14. Procédé selon la revendication 13, **caractérisé en ce qu'**une matière synthétique à base de méthacrylate photodurcissable est employée pour fabriquer le modèle en matière synthétique.

15. Procédé selon la revendication 13 ou 14, **caractérisé en ce que** la masse réfractaire est une masse d'intégration à base de quartz ou de cristobalite.

16. Procédé selon l'une quelconque es revendications 13 à 15, **caractérisé en ce que** la masse réfractaire est cuite avec le modèle intégré en présence d'air.

17. Procédé selon l'une quelconque des revendications 13 à 16, **caractérisé en ce que** l'opération de cuisson est effectuée à une température supérieure ou égale à 800 °C, pouvant augmenter jusqu'à 1000 °C.

18. Procédé selon l'une quelconque des revendications 13 à 17, **caractérisé en ce que** la masse de lithium-disilicate obtenue est introduite par pression dans la masse réfractaire sous vide à une température supérieure à 900 °C.

19. Procédé selon l'une quelconque des revendications 13 à 18, **caractérisé en ce que** l'implant démoulé est dépourvu de masse réfractaire adhésive par décapage avec un acide fluorhydrique dilué.
